# EUROPÄISCHE PATENTSCHRIFT

(11) **EP 1 737 880 B1**
(45) Veröffentlichungstag und Bekanntmachung des Hinweises auf die Patenterteilung: **14.04.2010**
(21) Anmeldenummer: 05732355.2
(22) Anmeldetag: 15.04.2005
(51) Int. Cl.: C07J 41/00, A61K 31/56, A61P 5/28

(54) **17 ALPHA-FLUOR 17 BETA-HYDROXIMINOMETHYL-STEROIDE, EIN VERFAHREN ZU DEREN HERSTELLUNG UND DIESE VERBINDUNG ENTHALTENDE PHARMAZEUTISCHE ZUSAMMENSETZUNGEN**
17ALPHA-FLUORO-17BETA-HYDROXIMINOMETHYL STEROIDS, A METHOD FOR PRODUCTION THEREOF AND PHARMACEUTICAL COMPOSITIONS COMPRISING SAID COMPOUNDS
17ALPHA-FLUORO-17BETA-HYDROXIMINOMETHYL-STEROIDES, LEUR PROCEDE DE PRODUCTION, ET COMPOSITIONS PHARMACEUTIQUES LES CONTENANT

(30) Priorität: 19.04.2004 DE 102004019406
(43) Veröffentlichungstag der Anmeldung: 03.01.2007
(73) Patentinhaber: Bayer Schering Pharma Aktiengesellschaft, 13353 Berlin (DE)
(72) Erfinder: BOTHE, Ulrich, 10551 Berlin (DE); MENZENBACH, Bernd, 07743 Jena (DE); DROESCHER, Peter, 99425 Weimar (DE); SCHWEIKERT, Hans-Udo, 53125 Bonn (DE); ELGER, Walter, 14195 Berlin (DE); HILLISCH, Alexander, 42553 Velbert (DE); REDDERSEN, Gudrun, 07749 Jena (DE); SCHNEIDER, Birgit, 07745 Jena (DE)
(86) Internationale Anmeldenummer: PCT/EP2005/003977
(87) Internationale Veröffentlichungsnummer: WO 2005/100376

(56) Entgegenhaltungen:
- REICHERT WOLFGANG ET AL: "Stable expression of human 5alpha-reductase type II in COS1 cells due to chromosomal gene integration: A novel tool for inhibitor identification" JOURNAL OF STEROID BIOCHEMISTRY AND MOLECULAR BIOLOGY, Bd. 78, Nr. 3, September 2001 (2001-09), Seiten 275-284, XP002329766 ISSN: 0960-0760
- HARTMANN ROLF W ET AL: "Synthesis and evaluation of novel steroidal oxime inhibitors of P450 17 (17alpha-hydroxylase/C17-20-Lyase) and 5alpha-reductase types 1 and 2" JOURNAL OF MEDICINAL CHEMISTRY, Bd. 43, Nr. 22, 2. November 2000 (2000-11-02), Seiten 4266-4277, XP002329767 ISSN: 0022-2623
- PETROW V ET AL: "PROSTATIC CANCER 2. INHIBITORS OF RAT PROSTATIC 4 ENE-3 KETO STEROID 5-ALPHA REDUCTASE DERIVED FROM 6 METHYLENE-4 ANDROSTEN-3-ONES" JOURNAL OF STEROID BIOCHEMISTRY, Bd. 19, Nr. 4, 1983, Seiten 1491-1502, XP002329768 ISSN: 0022-4731
- PETROW V ET AL: "PROSTATE III A STRUCTURAL FEATURE CHARACTERISTIC OF THE RAT PROSTATE 5-ALPHA REDUCTASE ACTIVE SITE" JOURNAL OF STEROID BIOCHEMISTRY, Bd. 32, Nr. 3, 1989, Seiten 399-408, XP002329769 ISSN: 0022-4731

## Beschreibung

### Technisches Gebiet

Die Erfindung betrifft 17α-Fluorsteroide, ein Verfahren zu deren Herstellung und diese Verbindungen enthaltende pharmazeutische Zusammensetzungen.

Die erfindungsgemäßen Verbindungen besitzen ein Wirkprofil mit Hybridcharakter in der Weise, dass sie als Inhibitoren der 5α-Reduktase und gleichzeitig als Gestagene wirken. Sie eignen sich daher zur Behandlung von Erkrankungen, die bei Mann und Frau in bestimmten Organen und Geweben Folge erhöhter Androgenspiegel sind, z.B. Akne, Hirsutismus, Alopezie (männlicher Typ), BPH und Prostatakarzinom. In Kombination mit anderen hormonalen Substanzen, wie einem Östrogen, Testosteron bzw. einem starken Androgen, sind die erfindungsgemäßen Verbindungen geeignet als Kontrazeptiva für die Frau und für den Mann.

### Stand der Technik

Aus der Patentliteratur - DE 100 43 846.6 sind 17-Chlormethylen-Verbindungen bekannt, die zwar die 5α-Reduktase hemmen, jedoch nur schwach gestagen im Vergleich zum natürlichen Progesteron sind, wie anhand von Untersuchungen zur Schwangerschaftserhaltung mit diesen Verbindungen klar demonstriert wird.

J. St. Biochem. Mol. Biol. 78(3), 275-84 und J. Med. Chem. 43(22), 4266-77 beschreiben Progesteron-20-oxim als Hemmer von 5α-Reduktase.

### Darstellung der Erfindung

Der vorliegenden Erfindung liegt somit die Aufgabe zugrunde, Verbindungen zu finden, die gegenüber den aus dem Stand der Technik bekannten Verbindungen ein umfassenderes Wirkprofil besitzen.

Erfindungsgemäß wird die Aufgabe durch vorgenannte 17α-Fluorsteroide nach Formel I 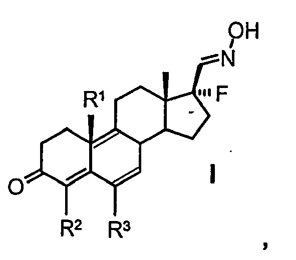 gelöst, worin R¹, falls anwesend, ein Wasserstoffatom oder eine Methylgruppe ist. R² und R³ sind ein Wasserstoffatom, ein Chloratom oder eine Methylgruppe und zwischen den Kohlenstoffatomen 6 und 7 kann sich eine Einfach- oder eine Doppelbindung befinden und zwischen den Kohlenstoffatomen 9 und 10 kann sich eine Einfach- oder eine Doppelbindung befinden kann.

Eine bevorzugte Ausführungsform der Erfindung ist die im Patentanspruch 2 aufgezeigte Verbindung (E)-17α-Fluor-3-oxo-estr-4-en-17β-carbaldehyd oxim nach Formel II

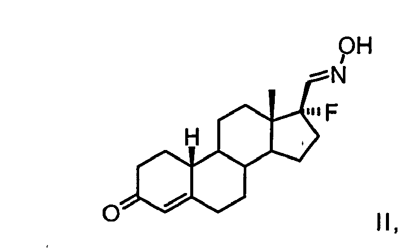

Die erfindungsgemäßen Verbindungen nach Anspruch 1 sind zur Herstellung pharmazeutischer Zusammensetzungen und Zubereitungen geeignet. Die pharmazeutischen Zusammensetzungen beziehungsweise Arzneimittel enthalten als Wirkstoffe die erfindungsgemäßen 17α-Fluorsteroide, gegebenenfalls in Mischung mit anderen pharmakologisch beziehungsweise pharmazeutisch wirksamen Stoffen und gegebenenfalls zusammen mit pharmazeutisch verträglichen Hilfs- und Trägerstoffen.

Eine vorteilhafte Ausgestaltung der Erfindung besteht in der Verwendung der erfindungsgemäßen 17α-Fluorsteroide zur Herstellung eines Arzneimittels zur Behandlung von Prostataerkrankungen, Alopezie vom männlichen Typ, Akne und Hirsutismus, zur Kontrazeption bei Mann und Frau und/oder zur Inhibition der 5α-Reduktase und zur Behandlung von Krebserkrankungen, die durch Androgene ungünstig beeinflusst werden, beispielsweise Prostatakarzinom.

Die mit der Erfindung erzielten Vorteile bestehen insbesondere darin, dass überraschenderweise strukturell neuartige 17α-Fluorsteroide gefunden wurden, die sowohl als signifikante Inhibitoren der 5α-Reduktase als auch als hochpotente Gestagene wirken.

Die erfindungsgemäßen Verbindungen eignen sich infolge ihres Wirkprofils mit Hybridcharakter daher zur Behandlung von Erkrankungen, die in bestimmten Organen und Geweben bei Mann und Frau Folge erhöhter Androgenspiegel sind, wie Akne, Hirsutismus, Alopezie (männlicher Typ), BPH und Prostatakarzinom. Bei der Frau können erhöhte Spiegel an 5α-Dihydroprogesteron zu schweren Störungen der Befindlichkeit beim prä menstruellen Syndrom beitragen. Dieses kann durch Hemmung der 5α-Reduktase günstig beeinflusst werden.

Das gleichzeitige Vorliegen einer gestagenen Wirkung bei den erfindungsgemäßen Verbindungen führt beim männlichen und weiblichen Geschlecht zu einer Hemmung der Gonadenfunktion. Dieser Effekt ist dann erwünscht, wenn mit der Behandlung eine antifertile Wirkung oder auch eine Hemmung der Hormonsekretion der Gonade erreicht werden soll. Dies ist bei Erkrankungen der Prostata (benigne Prostatahyperplasie) häufig der Fall. Mögliche Indikationen sind neben Prostataerkrankungen die Kontrazeption bei beiden Geschlechtern, Alopezie vom männlichen Typ, Akne und Hirsutismus und die Behandlung von Krebserkrankungen, die von Androgenen ungünstig beeinflußt werden (Prostatakarzinom).

In Verbindung mit anderen hormonalen Substanzen, wie einem Östrogen, Testosteron oder Testosteronester bzw. einem starken Androgen, sind die erfindungsgemäßen Verbindungen geeignet als Kontrazeptiva für die Frau bzw. für den Mann. Im letzteren Fall wird die Wirkung von Testosteron in der Prostata durch Hemmung der 5α-Reduktase abgeschwächt, während die gestagene Aktivität die Wirkung in der männlichen Gonade, das heißt die Hemmung der Spermatogenese, verstärkt.

Werden die erfindungsgemäßen Verbindungen für die weibliche Kontrazeption eingesetzt, können sie allein oder in Verbindung mit einem Estrogen verwendet werden. Als Estrogene sind grundsätzlich alle estrogenwirksamen Verbindungen geeignet: Estrogene, die verwendet werden können, sind beispielsweise Ethinylestradiol, 17β-Estradiol sowie dessen Ester, wie Estradiol-3-benzoat, Estradiol-17-valerat, -cypionat, - undecylat, -enanthat und/oder weitere Estradiolester und konjugierte Estrogene und Prodrugs von Estradiol und anderen natürlichen Estrogenen, z.B. Estradiol-3-sulfamat.
Werden die erfindungsgemäßen Verbindungen für die männliche Kontrazeption eingesetzt, können sie allein oder in Verbindung mit einem Androgen gegebenenfalls mit Testosteron und/oder Testosteronestern, verwendet werden.

Die Herstellung der Arzneimittel erfolgt in bekannter Weise, wobei die aus der Literatur bekannten und üblichen pharmazeutischen Hilfs- und Trägerstoffe für Pharmazie, Kosmetik und angrenzende Gebiete verwendet werden können.

Die erfindungsgemäßen Verbindungen nach Anspruch 1 können oral als Kapseln, Pillen, Tabletten, Dragees usw. oder parenteral, beispielsweise intraperitoneal, intramuskulär, subkutan oder perkutan, verabreicht werden.

Die Verbindungen können auch in das Gewebe implantiert werden. Die Dosierungseinheiten können neben dem Wirkstoff einen pharmazeutisch verträglichen Träger, wie zum Beispiel Stärke, Zucker, Sorbit, Gelatine, Gleitmittel, Kieselsäure, Talkum usw., enthalten.

Zur parenteralen Verabreichung kann der Wirkstoff in einem physiologisch verträglichen Verdünnungsmittel gelöst oder suspendiert sein. Als Verdünnungsmittel werden sehr häufig Öle mit oder ohne Zusatz eines Lösungsvermittlers, eines oberflächenaktiven Mittels, eines Suspendier- oder Emulgiermittels verwendet. Beispiele für verwendete Öle sind Olivenöl, Erdnussöl, Baumwollsamenöl. Sojabohnenöl, Rizinusöl und Sesamöl.

Die erfindungsgemäßen Verbindungen nach Anspruch 1 lassen sich auch in Form einer Depotinjektion oder eines Implantatpräparats anwenden, die so formuliert sein können, dass eine verzögerte Wirkstoff-Freigabe ermöglicht wird. Implantate können als inerte Materialien zum Beispiel biologisch abbaubare Polymere enthalten oder synthetische Silikone wie zum Beispiel Silikonkautschuk.

Der Wirkstoff kann außerdem zur perkutanen Applikation zum Beispiel in ein Pflaster eingearbeitet werden.

Für die Herstellung von mit den erfindungsgemäßen Verbindungen beladenen Intravaginal- (z. B. Vaginalringe) oder Intrauterinsystemen (z. B. Pessare, Spiralen, IUSs) für die lokale Verabreichung eignen sich verschiedene Polymere wie zum Beispiel Silikonpolymere, Ethylenvinylacetat, Polyethylen oder Polypropylen.

Um eine bessere Bioverfügbarkeit des Wirkstoffes zu erreichen, können die Verbindungen auch als Cyclodextrinclathrat formuliert werden. Hierzu werden die Verbindungen mit α-, β- oder γ-Cyclodextrin oder Derivaten von diesen umgesetzt.

Erfindungsgemäß können die Verbindungen der allgemeinen Formel I auch mit Liposomen verkapselt werden.

Gegenstand der vorliegenden Erfindung ist auch die Verwendung der 17α-Fluorsteroide nach Anspruch 1 als therapeutische Wirkstoffe besonders zur Behandlung von Prostataerkrankungen, Alopezie vom männlichen Typ, Akne und Hirsutismus, zur Kontrazeption bei Mann und Frau und/oder zur Inhibition der 5α-Reduktase und zur Behandlung von Krebserkrankungen, die durch Androgene ungünstig beeinflusst werden, z.B. Prostatakarzinom.

### Ausführungsbeispiel

Die Herstellung und die physikalischen Eigenschaften der erfindungsgemäßen 17-α-Fluorsteroide nach Anspruch 1 wird im folgenden beispielhaft an der Synthese der besonders bevorzugten Verbindung (E)17α-Fluor-3-oxo-estr-4-en-17β-carbaldehyd oxim erläutert.

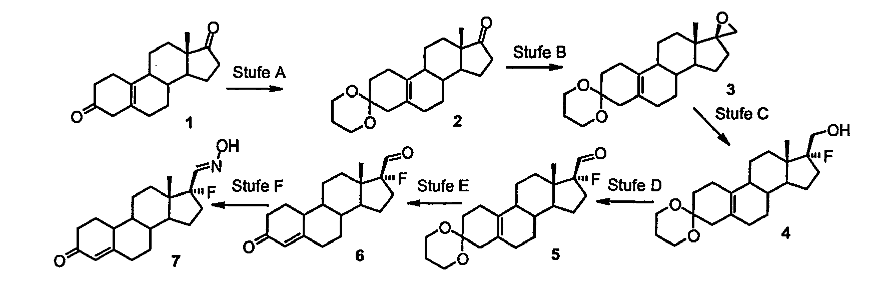

### Synthese von (E)-17α-Fluor-3-oxo-estr-4-en-17β-carbaldehyd oxim - hier 7

### Stufe A:

Es wird eine Mischung aus Estr-5(10)-en-3,17-dion (CAS-Nummer 3962-66-1, 15.1 g) 1 und Pyridinium-toluol-4-sulfonat PPTS (1.57 g) in 1,3-Propandiol (200 ml) 20 h bei Raumtemperatur unter Argon gerührt. Danach gießt man die Mischung auf gesättigte wässrige NaHCO₃-Lösung und saugt den ausgefallenen Feststoff ab. Der Feststoff wird zweimal mit Toluol versetzt und das Toluol am Rotationsverdampfer entfernt. Nach Trocknen im Vakuum erhält man 3-(1',3'-Dioxan-2'-yl)-estr-5(10)-en-17-on 2 als Feststoff (17.6 g). 1H-NMR (400 MHz, CDCl₃, ausgewählte Daten): δ = 0.88 (s, 3H, Me), 2.28 - 2.38 (m, 2H), 2.46 (dd, J = 9.1, 7.8 Hz, 1H), 3.8 - 4.1 (m, 4H, OC*H*₂CH₂C*H*₂O).

### Stufe B:

Es wird eine Lösung der Verbindung 2 aus Stufe A (15.0 g) in Dimethylformamid (180 ml) mit einem Eisbad abgekühlt und Kalium-*tert*-butylalkoholat (KOtBu, 10.9 g) Portionsweise zugegeben. Die eiskalte Mischung wird 2 h unter Argon gerührt, auf gesättigte wässrige NH₄Cl-Lösung gegossen und mit Ethylacetat extrahiert. Danach wird mit Wasser und gesättigter Kochsalzlösung gewaschen und über Natriumsulfat getrocknet. Nach Einengen am Rotationsverdampfer erhält man (17S)-spiro-3-(1',3'-Dioxan-2'-yl)-estr-5(10)-en-17,2'-oxiran 3 als Rohprodukt (Kristallbrei, 16.2 g), das ohne weitere Reinigung weiter umgesetzt wird.
Ausgewählte analytische Daten des Rohproduktes **3:** 1H-NMR (400 MHz, CDCl₃): δ = 0.89 (s, 3H, Me), 2.61 (d, J = 5.1 Hz, 1H, C20H(a)), 2.92 (d, J = 5.1 Hz, 1H, C20H(b)), 3.87 - 4.06 (m, 4H, OC*H*₂CH₂C*H*₂O).

### Stufe C:

Es wird der Kristallbrei in Diisopropylethylamin (Hünig-Base, 80 ml) vorgelegt und *N*-Ethyldiisopropylamin-tris-hydrofluorid (50 ml) zugegeben. Nach 20 min Erwärmung unter Rückfluss, wird weiteres Diisopropylethylamin (50 ml) zugegeben und 4 h unter Rückfluss erwärmt. Danach kühlt man auf Raumtemperatur ab, gießt die Reaktionsmischung vorsichtig auf eiskalte gesättigte wässrige NaHCO₃-Lösung und extrahiert mit Dichlormethan. Die organische Phase wird mit Wasser und gesättigter Kochsalzlösung gewaschen und eingeengt. Nach säulenchromatographischer Reinigung an Kieselgel erhält man eine Mischfraktion (3.0 g) aus 3-(1',3'-Dioxan-2'-yl)-17α-fluor-17β-hydroxymethyl-estr-4-en (Mindermengenkomponente **4**), 3-(1',3'-Dioxan-2'-yl)-17β-fluormethyl-17α-hydroxy-estr-4-en (Hauptmengenkomponente) und anderen, nicht näher identifizierten Komponenten.
19F-NMR (376 MHz, CDCl₃): δ = -223.1 (t, *J* = 49 Hz, CH₂F, Hauptmengenkomponente); -162.2 - -161.8 (m, C-17 Fluoratom, Mindermengenkomponente **4**).

### Stufe D:

Die Mischfraktion aus Stufe C wird in Dichlormethan (35 ml) gelöst und unter Argon Dess-Martin-Perjodinan (1,1-Dihydro-1,1,1-triacetoxy-1,2-benzjod-oxol-3(1H)-on, 1.43 g) zugegeben. Nach 75 min Rühren bei Raumtemperatur, wäscht man mit 10%iger wässriger Natriumthiosulfat-Lösung, gesättigter NaHCO₃- und Kochsalzlösung. Nach Trocknen über Natriumsulfat wird am Rotationsverdampfer eingeengt und säulenchromatographisch an Kieselgel (Laufmittel Toluol / Ethylacetat 15:1) gereinigt. Man erhält einen zähen Schaum (729 mg), der 3-(1',3'-Dioxan-2'-yl)-17α-fluor-estr-4-en-17β-carbaldehyd 5 als Hauptkomponente enthält. 1H-NMR (400 MHz, CDCl₃, ausgewählte Daten): δ = 0.77 (s, 3H, Methyl), 3.87 - 4.04 (m, 4H, OC*H*₂CH₂C*H*₂O, 9.77 (d, *J*(H,F) = 6.3 Hz, 1H, CH=O); 19F-NMR (376 MHz, CDCl₃): δ = -164.1 - -163.9 (m, symmetrisch).

### Stufe E:

Es wird eine Mischung des Schaums (719 mg) aus Stufe D und *p-*Toluolsulfonsäure Hydrat (230 mg) in Aceton (12 ml) 5 h 20 min bei Raumtemperatur unter Argon gerührt, mit gesättigter wässriger NaHCO₃-Lösung verdünnt, mit Ethylacetat extrahiert, mit gesättigter Kochsalzlösung gewaschen und mit Natriumsulfat getrocknet. Nach zweimaliger säulenchromatographischer Reinigung an Kieselgel erhält man 17α-Fluor-3-oxo-estr-4-en-17β-carbaldehyd 6 als Rohprodukt. 1H-NMR (400 MHz, CDCl₃, ausgewählte Daten): δ = 5.84 (s, 1H, 4-H), 9.78 (d, J(H,F) = 5.8 Hz, 1H, CHO).

### Stufe F:

Das Rohprodukt aus Stufe E (170 mg) wird in Dichlormethan (2 ml) und Pyridin (0.5 ml) gelöst und mit einem Eiskältebad abgekühlt. Man gibt Hydroxylaminhydrochlorid (23.3 mg) zu und rührt die eiskalte Lösung 1 h unter Argon. Es wird Wasser zugegeben, mit Dichlormethan und zweimal mit wässriger Salzsäurelösung (0.5 M) gewaschen. Es werden die Waschphasen mit Dichlormethan extrahiert und die vereinigten organischen Phasen mit Wasser und gesättigter Kochsalzlösung gewaschen. Nach Trocknen über Natriumsulfat engt man am Rotationsverdampfer ein und erhält einen Schaum, der säulenchromatographisch an Kieselgel gereinigt wird. Nach Kristallisation aus Cyclohexan / Aceton erhält man (E)-17α-Fluor-3-oxo-estr-4-en-17β-carbaldehyd oxim - hier 7 als weißen Feststoff (46 mg). C₁₉H₂₆FNO₂ (319.4). 1H-NMR (400 MHz, CDCl₃): δ = 0.75 (s, 3H, Methyl), 1.2 - 2.6 (m, 20H), 5.84 (s, 1H, 4-H), 7.32 (br. s, 1H, N=OH), 7.51 (d, *J* = 9.0 Hz, 1H, CH=N). 19F-NMR (376 MHz, CDCl₃): δ = -151.1 - -151.0 (m, symmetrisch).

Nachfolgend sind beispielhafte Daten zur Progesteronrezeptorbindung, 5α-Reduktase-Typ2-Aktivität in Genitalhaut-Homogenaten und in vivo- Daten am Beispiel von (E)-17α-Fluor-3-oxo-estr-4-en-17β-carbaldehyd oxim aufgeführt

### Bindung der Verbindung I an den Progesteronrezeptor

| | |
|---|---|
| Biol. Material: | Uterus-Cytosol, Kaninchen, geprimt |
| Tracer: | ³H-ORG 2058/Inkubationsbedingungen 0-4°C; 18 h |
| Referenzsubstanz: | Progesteron = 100% |
| Relative Bindungsaffinität RBA[%]: | 131 ± 9% |

| | |
|---|---|
| **5**α**- Reduktase-Typ2-Aktivität** | |

In Genitalhaut-Homogenaten unter optimierten Bedingungen bei pH 5.5: Verbindung 7: IC₅₀ = 245 nM.

### PR-B Antagonismus und Agonismus

Antagonismus IC₅₀ (mol/L) = 3.39E-10
Agonismus IC₅₀ (mol/L) = 2.5E-10

### In vivo Daten

| **Gestagene Aktivität im Schwangerschaftserhaltungstest Maus** | | |
|---|---|---|
| | **Dosis (mg/Tier/Tag)** | **Rate der erhaltenen Graviditäten** |
| 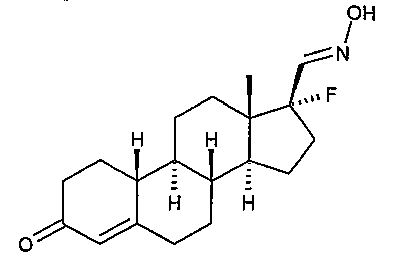 | 0,003 | 0/3 |
| | | |
| | 0,03 | 2/2 |
| | | |
| | 0,3 | 3/3 |

Die oben aufgeführten Daten belegen das Wirkprofil mit Hybridcharakter der erfindungsgemäßen Verbindungen, die als Inhibitoren der 5α-Reduktase und als Gestagene wirken.

## Patentansprüche

1. 17α-Fluorsteroide der allgemeinen Formel I 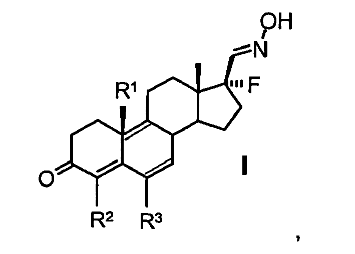 worin
R¹, falls anwesend, ein Wasserstoffatom oder eine Methylgruppe ist,
R2 und R3 ein Wasserstoffatom, ein Chloratom oder eine Methylgruppe sind und
zwischen den Kohlenstoffatomen 6 und 7 sich eine Einfach- oder eine Doppelbindung befinden kann und
zwischen den Kohlenstoffatomen 9 und 10 sich eine Einfach- oder eine Doppelbindung befinden kann.

2. 17α-Fluorsteroide nach Anspruch 1, nämlich
(E)-17α-Fluor-3-oxo-estr-4-en-17β-carbaldehyd oxim

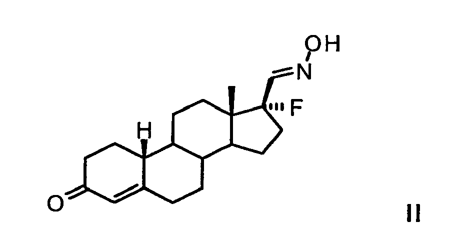

3. Verwendung der 17α-Fluorsteroide der Formel I zur Herstellung eines Arzneimittels zur Behandlung von Prostataerkrankungen, Alopecie vom männlichen Typ, Akne und Hirsutismus, zur Kontrazeption bei Mann und Frau und/oder zur Inhibition der 5α-Reduktase und zur Behandlung von Krebserkrankungen, die durch Androgene ungünstig beeinflusst werden, beispielsweise Prostatakarzinom.

## Claims

1. 17α-fluorosteroids of the general formula I 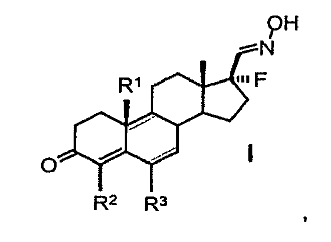 in which
R¹, if present, is a hydrogen atom or a methyl group,
R² and R³ are a hydrogen atom, a chlorine atom or a methyl group, and
there may be a single or a double bond between carbon atoms 6 and 7, and
there may be a single or a double bond between carbon atoms 9 and 10.

2. 17α-fluorosteroids according to Claim 1, namely (E)-17α-fluoro-3-oxoestr-4-ene-17β-carbaldehyde oxime

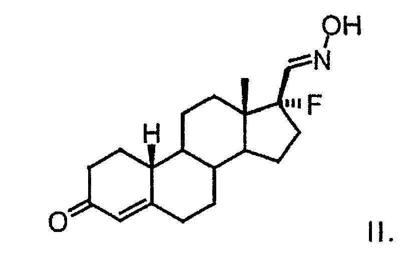

3. Use of the 17α-fluorosteroids of the formula I for producing a medicament for the treatment of prostate disorders, male-type alopecia, acne and hirsutism, for male and female contraception and/or for inhibiting 5α-reductase and for treating cancers which are unfavourably influenced by androgens, for example prostate carcinoma.

## Revendications

1. 17α-fluorostéroïdes de formule générale I 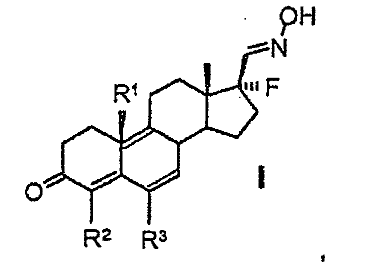 dans lesquels
R¹, s'il est présent, est un atome d'hydrogène ou un groupe méthyle,
R² et R³ représentent chacun un atome d'hydrogène, un atome de chlore ou un groupe méthyle, et
une liaison simple ou une double liaison peut se trouver entre les atomes de carbone 6 et 7, et
une liaison simple ou une double liaison peut se trouver entre les atomes de carbone 9 et 10.

2. 17α-fluorostéroïdes selon la revendication 1, plus précisément (E)-17 α-fluoro-3-oxo-oestr-4-ène-17β-carbaldéhyde-oxime

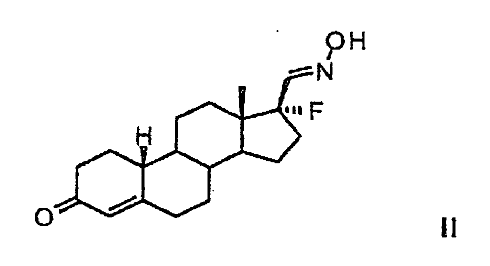

3. Utilisation des 17α-fluorostéroïdes de formule I pour la préparation d'un médicament destiné au traitement de maladies de la prostate, de l'alopécie hippocratique, de l'acné et de l'hirsutisme, à la contraception chez l'homme et la femme et/ou à l'inhibition de la 5α-réductase, ou au traitement de maladies cancéreuses qui sont influencées d'une manière défavorable par les androgènes, par exemple le cancer de la prostate.
